# EUROPEAN PATENT SPECIFICATION

(11) **EP 2 482 658 B1**
(45) Date of publication and mention of the grant of the patent: **01.07.2015**
(21) Application number: 10821290.3
(22) Date of filing: 30.09.2010
(51) Int. Cl.: A61K 9/14, A61K 9/72, A61P 9/00, A61K 31/4422

(54) **METHODS AND COMPOSITIONS FOR TREATMENT OF RAYNAUD'S PHENOMENON**
VERFAHREN UND ZUSAMMENSETZUNGEN ZUR BEHANDLUNG DES RAYNAUD-SYNDROMS
PROCÉDÉS ET COMPOSITIONS POUR LE TRAITEMENT DU PHÉNOMÈNE DE RAYNAUD

(30) Priority: 30.09.2009 US 247323 P
(43) Date of publication of application: 08.08.2012
(73) Proprietor: MicroDose Therapeutx, Inc., Monmouth Junction, NJ 08852 (US)
(72) Inventor: COOK, Robert, Hillsborough NJ 08844 (US)
(74) Representative: Cummings, Ross Martin
(86) International application number: PCT/US2010/050983
(87) International publication number: WO 2011/041595

(56) References cited:
- WO-A1-2008/156820
- WO-A1-2009/049242
- WO-A2-2012/030309
- US-A- 4 605 552
- US-A1- 2002 142 050
- US-A1- 2003 073 127
- BAKST R ET AL: "Raynaud's phenomenon: Pathogenesis and management", JOURNAL OF THE AMERICAN ACADEMY OF DERMATOLOGY, C.V. MOSBY, ST. LOUIS, MO, US, vol. 59, no. 4, 1 October 2008 (2008-10-01), pages 633-653, XP025470534, ISSN: 0190-9622, DOI: 10.1016/J.JAAD.2008.06.004 [retrieved on 2008-07-24]

## Description

The present invention relates generally to a method of treating Raynaud's Phenomenon and associated vascular disorders. Also disclosed are dosage forms containing rapid acting calcium channel blocking medications adapted for pulmonary route administration.

Raynaud's Phenomenon (RP) is a condition resulting in a particular series of discolorations of the fingers and/or the toes after exposure to changes in temperature (cold or hot) or stressful emotional events. Skin discoloration occurs because an abnormal spasm of the blood vessels causes a diminished blood supply to the local tissues. Initially, the digit(s) involved turn white because of the diminished blood supply. The digit(s) then turn blue because of prolonged lack of oxygen. Finally, the blood vessels reopen, causing a local "flushing" phenomenon, which turns the digit(s) red. This three-phase color sequence (white to blue to red) most often occurs upon exposure to cold temperature, is characteristic of RP and is accompanied with pain. Chronic recurrent cases of RP can, in some cases, result in atrophy of the skin, subcutaneous tissues, and muscle. RP can also cause ulceration and ischemic gangrene.

Raynaud's Phenomenon most frequently affects women, especially in the second, third, or fourth decades of life. People can have RP alone or as a part of other rheumatic diseases. When it occurs alone, it is referred to as "Raynaud's Disease" or primary Raynaud's Phenomenon. When it accompanies other diseases, it is called secondary Raynaud's Phenomenon.

Current therapies for RP require either (i) daily prophylactic medication delivered orally or intravenously, e.g. with a calcium channel blocker or B-blocker as will be discussed below, which is undesirable due to long term and frequent side effects (e.g. postural hypertension), or (ii) are slow in onset and unreliable for treating symptoms as they occur (acutely).

Calcium channel blockers (CCB's) are a class of drugs and natural substances which disrupt the conduction of calcium channels. Calcium channel blockers have effects on many excitable cells of the body, such as cardiac muscle, i.e. heart, smooth muscles of blood vessels, and neurons. The main clinical usage of calcium channel blockers is to decrease blood pressure. It is for this action that they are used in individuals with hypertension. Calcium channel blockers work by blocking voltage-gated calcium channels (VGCCs) in cardiac muscle and blood vessels. This decreases intracellular calcium leading to a reduction in muscle contraction. In the heart, a decrease in calcium available for each beat results in a decrease in cardiac contractility. In blood vessels, a decrease in calcium results in less contraction of the vascular smooth muscle and therefore an increase in arterial diameter (CCB's do not work on venous smooth muscle), a phenomenon called vasodilation. Vasodilation decreases total peripheral resistance, while a decrease in cardiac contractility decreases cardiac output. Since blood pressure is determined by cardiac output and peripheral resistance, blood pressure drops. With a relatively low blood pressure, the afterload on the heart decreases; this decreases the amount of oxygen required by the heart.

However, because calcium channel blockers result in a decrease in blood pressure, the baroreceptor reflex often initiates a reflexive increase in sympathetic activity leading to increased heart rate and contractility.

US 20020142050 describes the formulation of drugs with low aqueous solubility in a porous matrix form.

US 4,605,552 describes a method of treating angina pectoris by administering diltiazem by inhalation.

Bakst et al., J. Am. Acad. Dermatol, vol. 59, 4, pp 633-653 describes the pathogenesis and management of Raynaud's phenomenon.

Thus, there is a need for improved delivery of short acting calcium channel blockers for treating Raynaud's Phenomenon, which will provide rapid onset of activity, rapid bioavailability and minimized variations in blood levels, while at the same time providing relative ease of administration and reduced side effects compared to current delivery methods for administering a calcium channel blocker agent. Inhalation of a calcium channel blocker provides a method by which these agents can provide systemic drug levels for rapid onset of action and therapeutic relief without leading to the usual side effects, and potentially leading to a more rapid return to functional activity following an attack.

The foregoing and other objects of the invention are achieved by providing methods and compositions for pulmonary delivery of a calcium channel blocker to a mammalian host, particularly a human patient, whereby to provide for rapid absorption of a calcium channel blocker while avoiding the above and other disadvantages of conventional oral or intravenous administration.

More particularly, it has been discovered that a calcium channel blocker can be usefully administered to mammals in dry powder form, by pulmonary delivery to elicit a rapid systemic therapeutic response and provide enhanced bioavailability, and minimize variations in blood levels. Administration of a dry powder calcium channel blocker by pulmonary delivery provides a more rapid onset of activity, ease of administration, and reduced side effects as compared to conventional chronic oral method of administration. In a preferred embodiment of the invention, the calcium channel blocker comprises nicardipine in dry powder form for use by humans, although other calcium channel blockers that can be administered in dry powder form, including but not limited to clevidipine, also advantageously may be used.

As used herein, the term "a calcium channel blocker" or "CCB" is intended to encompass not only a calcium channel blocker in dry powder form, but any salt or derivative of a calcium channel blocker, in dry powder form, having calcium channel blocking activity, and which is non-toxic and pharmacologically acceptable.

"An effective amount," as used herein, is an amount of the pharmaceutical composition that is effective for treating Reynaud's Phenomenon, i.e., an amount of a calcium channel blocker of a defined particle size suitable for absorption in the lungs, that is able to reduce or eliminate the symptoms of Raynaud's Phenomenon.

"A pharmaceutical composition," as used herein, means a medicament for use in treating a mammal that comprises a calcium channel blocker in dry powder form of a defined particle size prepared in a manner that is suitable for pulmonary administration to a mammal. A pharmaceutical composition according to the invention may also, but does not of necessity, include a non-toxic pharmaceutically acceptable carrier.

"A defined particle size," as used herein, means particles having a size sufficiently small so as to be delivered to the lungs. For optimal delivery to the lungs, the dry powder form of the calcium channel blocker preferably should be micronized or spray dried to a median powder size of 0.5 - 10 µm (microns), preferably 1 - 6 µm (microns).

The dry powder form calcium channel blocker may then be put into a conventional dry powder inhaler (DPI) or metered dose inhaler (MDI) in a systemically effective unit dose delivery amount between about 0.1 and 16 milligrams of a calcium channel blocker as needed, typically 1 to 10 times per day. In a preferred embodiment of the invention, the dry powder form calcium channel blocker is packaged for delivery in a vibratory dry powder inhaler such as are available from MicroDose Therapeutx, Inc. of Monmouth Junction, New Jersey.

The dry powder delivery of calcium channel blocker to the respiratory tract can be used advantageously to treat acute or chronic Raynaud's Phenomonon symptoms. Unlike conventional intravenous delivery of calcium channel blockers which may require uncomfortable injections with potential significant side effects, dry powder delivery of a calcium channel blocker permits a patient to enjoy relief on an as-needed basis without painful needles.

The following examples are provided to further illustrate the present invention:

### Example 1

Clevidipine is an ultra-short acting calcium channel blocker with an onset of action of about 1-2 minutes. Currently, it is marketed only as an injectable emulsion as will be discussed below, and is indicated for the reduction of blood pressure, normally only when oral therapy is not feasible or not desirable.

Clevidipine, (butyroxymethyl methyl 4-(2',3'-dichlorophenyl)-2,6-dimethyl-1,4-dihydropyridine-3,5-dicarboxylate), has the structure depicted below:

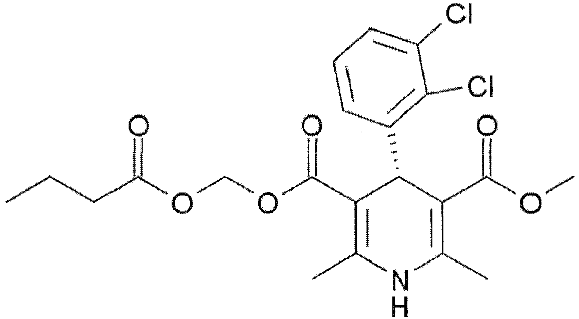

Clevidipine is a dihydropyridine L-type calcium channel blocker. It is highly selective for vascular, as opposed to myocardial, smooth muscle and, therefore, has little or no effect on myocardial contractility or cardiac conduction and is therefore likely to have a cleaner adverse effect profile. It reduces mean arterial blood pressure by decreasing systemic vascular resistance. Clevidipine is rapidly metabolized by esterases in the blood and extravascular tissues. Therefore, its elimination is unlikely to be affected by hepatic or renal dysfunction. Clevidipine does not accumulate in the body, and its clearance is independent of body weight.

The initial phase half-life is approximately 1 minute and the terminal half-life is approximately 15 minutes, providing an advantage of rapid onset of therapeutic action without longer term adverse effects. Clevidipine will still be rapidly metabolized in pseudocholinesterase-deficient patients.

Clevidipine is commercially formulated as a lipid emulsion in 20% soybean oil (Intralipid^{®}) and contains approximately 0.2 g of fat per mL (2.0 kcal/ml). Commercially formulated clevidipine also contains glycerin (22.5 mg/mL), purified egg yolk phospholipids (12 mg/mL), and sodium hydroxide to adjust pH.

Currently, clevidipine typically is administered by the intravenous route only, which, of course, requires uncomfortable injections into the patient. Also, as noted above, clevidipine contains several inactive ingredients, soybean oil, egg yolk, and phospholipids which are undesirable because some people may dislike or be allergic to one or more of these inactive ingredients that comprise the clevidipine injection.

Clevidipine in crystalline form is micronized by dry milling to a maximum particle size of about 1-10 µm (microns), and preferably 0.5-5 µm (microns). The powder is packaged for unit dose delivery of 2 milligrams in a dry powder inhaler (DPI) available from MicroDose Therapeutx, Inc.

### Example 2

Clevidipine dry powder as prepared in Example 1, and packaged for delivery in a DPI as before for delivery of 0.5 milligrams per dose.

### Conclusion

Delivery of 0.1-16 milligram doses of dry powder clevidipine delivered to the lungs, as needed, typically 1 -10 times per day, provides relief to patients suffering from Raynaud's Phenomenon.

### Example 3

Nicardipine is a calcium channel blocker with a rapid onset of action but with a slightly more extended action than clevidipine. Currently, it is marketed as the hydrochloride salt for oral capsule administration. Intravenous injection of nicardipine hydrochloride is also commercially available, and is indicated for the reduction of blood pressure, normally only when oral therapy is not feasible or not desirable.

Nicardipine hydrochloride is a dihydropyridine derivative with I U PAC (International Union of Pure and Applied Chemistry) chemical name (±)-2-(benzylmethyl amino) ethyl methyl 1,4-dihydro-2,6-dimethyl-4-(*m*-nitrophenyl)-3,5-pyridinedicarboxylate monohydrochloride and has the following structure:

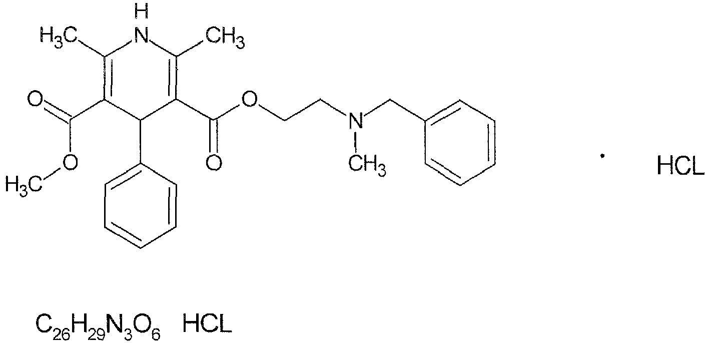

Nicardipine HCL is a calcium ion influx inhibitor (slow channel blocker). It is highly selective for vascular, as opposed to myocardial, smooth muscle and, therefore, has little or no effect on myocardial contractility or cardiac conduction and is therefore likely to have a cleaner adverse effect profile. It reduces mean arterial blood pressure by decreasing systemic vascular resistance. Following infusion, nicardipine plasma concentrations decline tri-exponentially. The initial phase half-life is approximately 2.7 minutes with a half-life of about 44.8 minutes, and a terminal half-life of approximately 14.4 hours, providing an advantage of rapid onset of therapeutic action, but with a longer term effect than clevidipine. Nicardipine does not accumulate in the body, and its clearance is independent of body weight.

Nicardipine is commercially formulated as a sterile, non-pyrogenic, clear, yellow solution in 10 mL ampuls for intravenous infusion after dilution. Each mL contains 2.5 mg nicardipine hydrochloride in Water for Injection, USP, with 48.00 mg Sorbitol, NF, buffered to pH 3.5 with 0.525 mg citric acid monohydrate, USP, and 0.09 mg sodium hydroxide, NF. Additional citric acid and/or sodium hydroxide may be added to adjust pH.

Currently, nicardipine typically is administered by the intravenous route only, which, of course, requires uncomfortable and inconvenient injections into the patient.

Nicardipine in crystalline form is micronized by dry milling to a maximum particle size of about 1-10 µm (microns), and preferably 0.5-5 µm (microns).

The powder is packaged for unit dose delivery of 2 milligrams in a dry powder inhaler (DPI) available from MicroDose Therapeutx, Inc.

### Example 4

Nicardipine dry powder as prepared in Example 3, and packaged for delivery in a DPI as before for delivery of 0-10 milligrams per dose.

### Conclusion

Delivery of 0.1-16 milligram doses of dry powder nicardipine delivered to the lungs, as needed, typically 1-10 times per day, provides relief to patients suffering from Raynaud's Phenomenon.

While the invention has been described in detail herein in accordance with certain preferred embodiments thereof, many modifications and changes therein may be affected by those skilled in the art. For example, while clevidipine or nicardipine in crystalline form can be micronized by dry milling to dry powder, other processes for making dry powder form of clevidipine or nicardipine may be employed including, but not limited to, spray drying, jet milling, and the like.

## Claims

1. A calcium blocker in dry powder form for use in the treatment of Raynaud's Phenomenon, **characterized in that** a systemically effective amount of a calcium channel blocker in dry powder form is delivered directly to a patient's lungs.

2. The calcium blocker in dry powder form for use according to claim 1, **characterized in that** the calcium channel blocker comprises clevidipine or nicardipine.

3. The calcium blocker in dry powder form for use according to claim 1, **characterized in that** the systematically effective amount of the calcium channel blocker is in unit dose amounts of between 0.1 and 50 milligrams.

4. The calcium blocker in dry powder form for use according to claim 3, **characterized in that** the systematically effective amount is for delivery at between 1 and 10 unit dose amounts per day.

5. The calcium blocker in dry powder form for use according to claim 1, **characterized in that** the dry powder calcium channel blocker has a median powder size of 0.5 to 10 µm, preferably a median particle size of 0.5 to 5 µm.

6. The calcium blocker in dry powder form for use according to claim 1, **characterized in that** the calcium channel blocker in dry powder form is delivered using a dry powder inhaler (DPI) or a metered dose inhaler (MDI).

7. The calcium blocker in dry powder form for use according to claim 6, **characterized in that** the calcium channel blocker in dry powder form is for delivery in a vibratory dry powder inhaler.

## Patentansprüche

1. Ein Calziumblocker in Trockenpulverform zur Verwendung bei der Behandlung des Raynaud-Phänomens, **dadurch gekennzeichnet, dass** eine systemisch wirksame Menge eines Calciumkanalblockers in Trockenpulverform direkt in die Lunge eines Patienten geleitet wird.

2. Der Calciumblocker in Trockenpulverform zur Verwendung nach Anspruch 1, bei dem der Calciumkanalblocker Clevidipin oder Nicardipin enthält.

3. Der Calciumblocker in Trockenpulverform zur Verwendung nach Anspruch 1, bei dem die systemisch wirksame Menge des Calciumkanalblockers in Einzeldosismengen zwischen 0,1 und 50 Milligramm vorliegt.

4. Der Calciumblocker in Trockenpulverform zur Verwendung nach Anspruch 3, bei dem die systemisch wirksame Menge zur Verabreichung von zwischen 1 und 10 Einzeldosismengen pro Tag vorliegt.

5. Der Calciumblocker in Trockenpulverform zur Verwendung nach Anspruch 1, bei dem der Trockenpulver-Calciumkanalblocker eine mittlere Pulverkörnung von 0,5 bis 10 µm, vorzugsweise eine mittlere Partikelgröße von 0,5 bis 5 µm hat.

6. Der Calciumblocker in Trockenpulverform zur Verwendung nach Anspruch 1, bei dem der Trockenpulver-Calciumkanalblocker mittels eines Trockenpulverinhalators (DPI) oder eines Dosieraerosols (MDI) verabreicht wird.

7. Der Calciumblocker in Trockenpulverform zur Verwendung nach Anspruch 6, bei dem der Trockenpulver-Calciumkanalblocker mittels eines Schwingungs-Trockenpulverinhalators verabreicht wird.

## Revendications

1. Un inhibiteur calcique sous forme de poudre sèche destiné à être utilisé pour le traitement du phénomène de Raynaud, **caractérisé en ce qu'**une quantité systématiquement efficace d'un inhibiteur des canaux calciques sous forme de poudre sèche est administrée directement dans les poumons d'un patient.

2. L'inhibiteur calcique sous forme de poudre sèche destiné à être utilisé selon la revendication 1, **caractérisé en ce que** l'inhibiteur des canaux calciques comprend de la clévidipine ou de la nicardipine.

3. L'inhibiteur calcique sous forme de poudre sèche destiné à être utilisé selon la revendication 1, **caractérisé en ce que** la quantité systématiquement efficace de l'inhibiteur des canaux calciques est présentée en quantités de doses unitaires comprises entre 0,1 et 50 milligrammes.

4. L'inhibiteur calcique sous forme de poudre sèche destiné à être utilisé selon la revendication 3, **caractérisé en ce que** la quantité systématiquement efficace est adaptée pour une administration du médicament comprise entre 1 et 10 quantités de doses unitaires par jour.

5. L'inhibiteur calcique sous forme de poudre sèche destiné à être utilisé selon la revendication 1, **caractérisé en ce que** l'inhibiteur des canaux calciques sous forme de poudre sèche présente une granulométrie moyenne de la poudre comprise entre 0,5 et 10 µm, de préférence une granulométrie moyenne des particules comprise entre 0,5 et 5 µm.

6. L'inhibiteur calcique sous forme de poudre sèche destiné à être utilisé selon la revendication 1, **caractérisé en ce que** l'inhibiteur des canaux calciques sous forme de poudre sèche est administré au moyen d'un inhalateur à poudre sèche (de type DPI) ou d'un aérosol-doseur (de type MDI).

7. L'inhibiteur calcique sous forme de poudre sèche destiné à être utilisé selon la revendication 6, **caractérisé en ce que** l'inhibiteur des canaux calciques sous forme de poudre sèche est conçu pour être administré dans un inhalateur à poudre sèche de type vibratoire.
